# EUROPEAN PATENT SPECIFICATION

(11) **EP 4 062 913 B1**
(45) Date of publication and mention of the grant of the patent: **08.05.2024**
(21) Application number: 21863656.1
(22) Date of filing: 02.09.2021
(51) Int. Cl.: A61K 31/427, A61K 9/20, A61K 9/22, A61K 9/50, A61K 47/30, A61P 35/00

(54) **SOLID ORAL FORMULATION OF UTIDELONE**
FESTE ORALE FORMULIERUNG VON UTIDELON
FORMULATION ORALE SOLIDE D'UTIDÉLONE

(30) Priority: 02.09.2020 CN 202010910072
(43) Date of publication of application: 28.09.2022
(73) Proprietor: Beijing Biostar Pharmaceuticals Co., Ltd., Beijing 101111 (CN); CHENGDU BIOSTAR PHARMACEUTICALS, LTD., Sichuan 611730 (CN)
(72) Inventor: TANG, Li, Beijing 101111 (CN); ZHANG, Chuan, Sichuan 611730 (CN); QIU, Rongguo, Beijing 101111 (CN)
(74) Representative: Høiberg P/S
(86) International application number: PCT/CN2021/116194
(87) International publication number: WO 2022/048592

(56) References cited:
- WO-A1-03/084536
- CN-A- 1 099 263
- CN-A- 1 496 258
- CN-A- 101 362 784
- CN-A- 104 666 297
- US-A1- 2006 134 214
- US-A1- 2019 070 152
- ELAINE T LAM ET AL: "Phase I dose escalation study of KOS-1584, a novel epothilone, in patients with advanced solid tumors", CANCER CHEMOTHERAPY AND PHARMACOLOGY, SPRINGER, BERLIN, DE, vol. 69, no. 2, 27 August 2011 (2011-08-27), pages 523-531, XP035006936, ISSN: 1432-0843, DOI: 10.1007/S00280-011-1724-7
- MA, Jingfan: "Made in Chengdu: The First Epothilone Type I Antitumor Drug Has Been Approved in China", Sichuan Science and Technology News, no. 17/03, 17 March 2021 (2021-03-17), pages 1-2, XP009534981, CN DOI: 10.28668/n.cnki.nsckj.2021.000098
- Guo, Lingyun; Fan Junfang: "Observation on the Phase II Trial of UTD1 Drugs in Stage IV Breast Cancer Patients", Chinese Nursing Research, vol. 29, no. 32, 30 November 2015 (2015-11-30), pages 4095-4097, XP009534942, CN ISSN: 1009-6493, DOI: 10.3969/j.issn.1009-6493.2015.32.051
- Zhang Pin, Tong Zhongsheng, Tian Fuguo, Wang Yongsheng, Yang Junlan, Li Weilian, Di Lijun, Liu Wei, Tang Li, Qiu Rongguo, Xu Bingh: "Phase II trial of utidelone as monotherapy or in combination with capecitabine in heavily pretreated metastatic breast cancer patients", Journal of Hematology & Oncology, vol. 9, no. 1, 1 December 2016 (2016-12-01), XP055907663, DOI: 10.1186/s13045-016-0297-7
- Zhang Pin, Sun Tao, Zhang Qingyuan, Yuan Zhongyu, Jiang Zefei, Wang Xiao, Cui Shude, Teng Yuee, Hu Xi-Chun, Yang Junlan, Pan Hongm: "Utidelone plus capecitabine versus capecitabine alone for heavily pretreated metastatic breast cancer refractory to anthracyclines and taxanes: a multicentre, open-label, superiority, phase 3, randomised controlled trial", Lancet oncology, vol. 18, no. 3, 1 March 2017 (2017-03-01), pages 371-383, XP009534943, AMSTERDAM, NL ISSN: 0140-6736, DOI: 10.1016/S1470-2045(17)30088-8
- Wang Jinxin, Gu Qin Lan, You Qi Dong , Sun Piao Yang: "Epothilones:A New Class of Antitumour Agent", Progress in Pharmaceutical Sciences, vol. 28, no. 8, 31 December 2004 (2004-12-31), pages 354-361, XP055907669,

## Description

### Technical Field

The present application belongs to pharmaceutical field, and specifically relates to a solid oral formulation of Utidelone and use thereof.

### Background Art

Utidelone is a class of epothilone derivatives belonging to macrolides and secondary metabolites produced by the genetically modified *Sorangium Cellulosum.* Studies have shown that epothilones have the same pharmacological mechanism as paclitaxel, which exerts antitumor effect by inhibiting the depolymerization of tubulin. The chemical name of Utidelone is: 4,8-dihydroxy-5,5,7,9,13-pentamethyl-16-[1-methyl-2-(2-methyl-thiazole-4-yl)-vinyl]-hexadecoxetane-13-en-2,6-one lactone with the chemical structure as shown below:

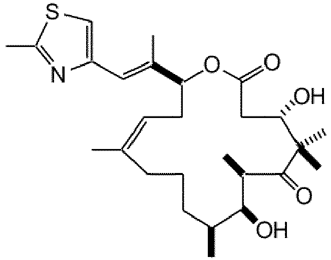

Utidelone injection (brand name: 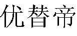 ^{™}), strength 5 ml: 50 mg, intravenous infusion for about 1.5 hours, dose 30-40 mg/m2/day, administered once a day for 5 consecutive days, 21 days as a treatment cycle, until disease progression or intolerable toxicity. Utidelone injection needs to be diluted with normal saline for injection (the final concentration of Utidelone is 0.2 mg/ml to 0.5 mg/ml) before use. It is used to treat patients with advanced breast cancer, lung cancer, gastric cancer, liver cancer and other solid tumors.

Utidelone is easily soluble in ethanol, methanol, ethyl acetate, and chloroform, but insoluble in water. The saturated solubility in water is less than 1 µg/ml, so it is difficult to develop into an oral formulation with suitable bioavailability. At present, the marketed epothilone antitumor drugs such as Utidelone injection and Ixabepilone injection etc., all of which use non-aqueous solvents such as ethanol and polyoxyl castor oil as solvents, are diluted with sodium chloride injection for administration by intravenous infusion. Since polyoxyl castor oil is a strong allergenic substance, antiallergic treatment must be given before intravenous administration, which reduces the compliance of this type of drug in clinical use, increases the adverse reactions of patients, and limits its clinical application.

Solid oral formulations of epothilone compounds are rare, and pharmaceutical formulations for intravenous injection are usually used for oral administration, such as those described in patent CN 101112373. Epothilone compounds are prone to ring-opening degradation in solution state and crystallize out due to poor solubility in the body, therefore the pharmaceutical formulations containing epothilone compounds in the form of solution for oral administration usually have poor stability, high irritation, and low bioavailability, are thus not pharmaceutically feasible. Therefore, it is an industry consensus to develop an oral formulation with high bioavailability and high drug stability.

Lam et al. 2011 (Cancer Chemother Pharmacol (2012), 69: 523-531) relates to a Phase I dose escalation study of the epothilone KOS-1584. KOS-1584 is administered intravenously in the study.

US 2006/134214 relates to enteric coated beads comprising epothilone or epothilone analogs, and a capsule comprising a multitude of such beads. The beads disclosed comprise enteric coating.

US 2019/070152 relates to pharmaceutical preparations comprising a de-epoxidized epothilone and use thereof in the treatment of tumors. The pharmaceutical preparations disclosed are liquid formulations.

### Summary

The invention is defined by the claims. Any subject matter falling outside the scope of the claims is provided for information purposes only.

In order to solve the above-mentioned problems, the present application provides an oral formulation using Utidelone as an active ingredient and a preparation method thereof. The oral formulation of the present application has high bioavailability and good physical and chemical stability, making it possible to administer such an active ingredient orally, improving medication compliance, and eliminating the strong allergic reaction caused by administering polyoxyl castor oil via injection.

The oral formulations of the present application not only effectively improves the solubility of Utidelone, but also solves the in vivo and in vitro stability of Utidelone, significantly improves the bioavailability of the formulation, and establishes a process for preparing the drug suitable for industrialized large-scale production. Moreover the dissolution and bioavailability of the solid oral formulations of the present application have been shown good bioavailability by the data.

According to one aspect, the present application provides a solid oral formulation comprising Utidelone, or a pharmaceutically acceptable salt, solvate or ester thereof as an active ingredient and a pharmaceutically acceptable excipient, wherein the formulation is a micropellet capsule encapsulating micropellets, or a tablet prepared by micropellet compression, wherein the micropellets are pellet cores coated with a coating layer comprising the active ingredient and said pharmaceutically acceptable excipient, wherein the pharmaceutically acceptable excipient comprises a hydrophilic pharmaceutical excipient, a sustained-release pharmaceutical excipient, and optionally a surfactant, and wherein the ratio of the active ingredient to the pharmaceutically acceptable excipient is in the range of 1:1 to 1:30.

In general, poorly soluble drugs may improve their solubility by reducing the particle size of API, preparing solid dispersions with hydrophilic carriers, and adding surfactants, etc. thereby improving the bioavailability of the drugs. Due to the extremely poor water solubility of Utidelone, it is difficult to obtain an ideal dissolution of the drug by the above mentioned general means such as reducing the particle size of the drug and adding surfactants. Therefore, it is very challenging and creative to obtain oral formulations with good physical and chemical stability and enhanced solubility and oral bioavailability of Utidelone.

The oral solid formulation containing Utidelone of the present application contains API (ie, active ingredient: Utidelone or a pharmaceutically acceptable salt, solvate or ester thereof) and pharmaceutical excipients. The oral formulation contains: 1) Utidelone or a pharmaceutically acceptable salt, solvate or ester thereof; 2) at least one hydrophilic pharmaceutical excipient; 3) at least one sustained-release pharmaceutical excipient; and 4) optional at least one surfactant.

The present application is not only applicable to Utidelone, but also to pharmaceutically acceptable salts, solvates or esters thereof.

According to some embodiments, the solid oral formulation of the present application, for example, in the form of a micropellet capsule, may contain: 1) Utidelone or a pharmaceutically acceptable salt, solvate or ester thereof; 2) at least one hydrophilic pharmaceutical excipient; 3) at least one sustained-release pharmaceutical excipient; and 4) at least one surfactant.

In the oral formulation of the present application, the ratio of Utidelone to the pharmaceutical excipients is in the range of 1:1 to 1:30, preferably 1:5 to 1:20.

The pellets in a micropellet capsule of the present application include a pill core (namely, pill cores) and a coating layer containing the drug. The pill core, for example, may be a circular or oval pharmaceutical excipient with a particle size of 100-1000 um. The scaffold material used for tablets or pill core is generally referred to as a pill core material, such as sucrose, starch, lactose, microcrystalline cellulose, mannitol and biodegradable polymers, etc.

The micropellet capsule or tablet contains about 2%-10% (w/w) of Utidelone, about 30%-70% (w/w) of pharmaceutical excipients, and about 20%-60% (w/w) pellet core material, calculated based on the total weight of pellets or tablets. Preferably, each capsule may contain 5-30 mg of Utidelone, and each tablet may contain about 5-20 mg of Utidelone.

The pill cores used in the preparation of micropellets for the oral formulation of the present application are selected from pill cores made of sucrose, starch, and microcrystalline cellulose ect., and sucrose pill core is preferable. The diameter of the pill core is 0.2 mm to 1.5 mm, preferably 0.4 mm to 1.0 mm. The particle size of the drug-coated pellets is 0.5-1.5 mm.

The hydrophilic pharmaceutical excipients in the oral formulations (such as micropellet capsules) of the present application are selected from one of povidone, hypromellose, mannitol, lactose, sucrose, poloxamer, polyvinyl alcohol, etc. or the mixtures thereof, for example, selected from low viscosity hypromellose, povidone and poloxamer.

The sustained-release pharmaceutical excipients in the oral formulations (such as micropellet capsules) of the present application are selected from one of povidone, hypromellose, polyethylene glycol, ethyl cellulose, polyvinyl acetal diethylamine acetate, hypromellose acetate succinate, acetate methacrylate copolymer, cellulose acetate, methyl cellulose, polyacrylic resin, polyvinyl phthalate, cellulose phthalate, and hypromellose phthalate, or the mixtures thereof, such as selected from the group consisting of high-viscosity hypromellose, high-viscosity polyethylene glycol, ethyl cellulose, and cellulose acetate.

The surfactant in the oral formulation of the present application (such as micropellet capsule) is selected from one of polysorbate, polyoxyl castor oil, sodium lauryl sulfate, cholate, fatty acid glyceride, sorbitan, polyoxyethylene fatty acid ester, polyoxyethylene fatty alcohol ether, and poloxamer, or the mixtures thereof. Preferred is polyoxyl castor oil, and second preferred is polysorbate or poloxamer.

According to one embodiment, the present application provides a solid oral formulation, such as a micropellet capsule, which uses Utidelone as the active ingredient and polyoxyethylene (40) hydrogenated castor oil, low-viscosity hypromellose (such as E50), and high-viscosity hypromellose (such as K100) as pharmaceutical excipients, and sucrose as the pill core.

Micropellet capsules containing Utidelone can be prepared as follows: dissolving Utidelone and the excipients in a solvent, which is then coated on pill cores to prepare micropellets, and finally encapsulated in capsules or compressed into tablets. This preparation method solves the problem of poor water solubility of Utidelone which results in poor drug bioavailability of the pharmaceutical preparation prepared by solid dispersion technology having the defects such as in vitro and in vivo recrystallization. Meanwhile, the oral formulation of the present application does not need to strictly control the particle size and crystal form of the API to ensure the stability of the preparation process. The dust generation in the production process is low, and it may be fully enclosed for preparation to reduce occupational hazards and other advantages. Utidelone exists in amorphous or molecular form in the oral solid preparation.

The above-mentioned oral formulation containing Utidelone and its preparation process have at least one of the following characteristics:
1) The oral formulation contains at least one hydrophilic pharmaceutical excipient to improve the solubility of the drug.
2) The oral formulation contains sustained-release pharmaceutical excipients to inhibit the release rate of the drug in the solid dispersion prepared with hydrophilic pharmaceutical excipients, thereby reducing the supersaturated concentration of the drug and inhibiting recrystallization of the drug in the body.
3) The oral formulation may contain one or more surfactants to further inhibit the recrystallization of the drug in vitro and in vivo, and also play a certain plasticizing role in the preparation process of pellets to increase the toughness of the finished micropellets.
4) During the preparation process, Utidelone needs to be first dissolved in an organic solvent or a mixture of organic solvents or a mixture of organic solvents and water.
5) During the preparation process, Utidelone dissolved in an organic solvent or a mixture of organic solvents or a mixture of organic solvents and water needs to be mixed with hydrophilic pharmaceutical excipients and/or sustained-release pharmaceutical excipients and/or surfactants, and optional other pharmaceutical excipients for co-drying to prepare a solid dispersion with higher solubility or micropellets with both higher solubility and sustained-release properties.

Above-mentioned organic solvent is a pharmaceutically acceptable organic solvent selected from the group consisting of ethanol, methanol, ethyl acetate, acetone, dichloromethane, chloroform, and the like. For example, ethanol, methanol, ethyl acetate or acetone, or a mixture of the above-mentioned two or more solvents mixed in any proportion.

The above-mentioned co-drying process is mainly fluidized bed coating process, spray drying process, reduced pressure vacuum drying process, heating drying, freeze drying and other drying processes.

The oral formulation of the present application, which is suitable for human patients (or animals), includes solid preparations such as soft and hard capsules, tablets, or micropellets such as micropellet capsules.

The daily dosage and frequency of administration may be based on commercially available unit formulations, and the daily dosage of the oral formulations of the present application may be obtained by administering half-unit, single unit, or more unit formulations

According to another aspect, the formulation of the present application is for use in a method of treatment of human or animal cancers. As used herein, cancers refer to various forms of cancers, and tumors refer to solid tumors, including breast cancer, lung tumors, digestive tract tumors, lymphoid tumors, prostate cancer, brain cancer, gynecological tumors, liver cancer, head and neck tumors, for example, breast cancer, lung cancer, liver cancer, ovarian cancer, colon cancer and stomach cancer.

### Definition of Terms

Pharmaceutical excipients refer to the excipients and additives used in formulating or producing medicines. They are substances other than active ingredients that have been reasonably evaluated in terms of safety and are included in pharmaceutical formulations.

Pill core (or called as pellet core) is a necessary starting master for the preparation of the matrix sustained release production.

Hydrophilic pharmaceutical excipients refer to pharmaceutical excipients that have a strong affinity for water, may attract water molecules, or are easily soluble in water. Sustained-release pharmaceutical excipients refer to the materials that may release the drug slowly to prolong the action time of the drug, which may be used to prepare medicaments, for example microcapsules, that may carry the drug and release it slowly.

Low-viscosity hypromellose refers to hypromellose with a viscosity of <80 mPa.S. For example, hypromellose E5, E3, E15, E50, K3, etc.

High-viscosity hypromellose refers to hypromellose with a viscosity of ≥80 mPa.S. For example, hypromellose K100LV, K100M, K100LVP, K4M, E4M, E4MP, K100MP, etc.

High-viscosity polyethylene glycol refers to polyethylene glycol with molecular weight of >1000, such as PEG1000, PEG2000, PEG4000, PEG8000, etc..

The present application has the following advantages;
1. The bioavailability of the oral formulation of the present application is high, even up to more than 55%.
2. The particle size and crystal form of the API do not need to be strictly controlled as in the preparation process of conventional oral solid formulations. After dissolving the active ingredient in a soluble organic solvent, it is sprayed onto the blank pill core through a coating process, and the Utidelone in the micropellet obtained after drying exists in the micropellet or powder in amorphous and molecular states and has a certain stability.
3. The preparation process of the oral formulation of the present application may adopt an integrated molding process. In the process of preparation, a closed operation is basically adopted, which may effectively avoid occupational exposure hazards caused by cytotoxic compounds.
4. The total yield of the oral formulation of the present application reaches more than 90%, which is far higher than the total yield of materials of conventional oral solid preparations.
5. The active ingredient of Utidelone in the solid oral formulation of the present application exists in an amorphous or molecular form, rather than in a crystalline form.

### Description of Drawings

**Figure 1****:** Dissolution profile of the oral formulation of Example 1;
**Figure 2****:** Dissolution profile of the oral formulation of Example 2;
**Figure 3****:** Dissolution profile of the oral formulation of Example 3;
**Figure 4****:** Dissolution profile of the oral formulation of Example 4;
**Figure 5A****:** Dissolution profile of the oral formulation of Example 5-A;
**Figure 5B****:** Dissolution profile of the oral formulation of Example 5-B;
**Figure 6****:** Area under the plasma concentration-time curve of Utidelone injection;
**Figure 7****:** Area under the plasma concentration-time curve of Utidelone capsules of Example 1;
**Figure 8****:** Area under the plasma concentration-time curve of Utidelone injection;
**Figure 9****:** Area under the plasma concentration-time curve of Utidelone capsules of Example 5-A;
**Figure 10****:** X-ray diffraction pattern of a crystal form of Utidelone;
**Figure 11****:** X-ray diffraction pattern of the amorphous form of Utidelone;

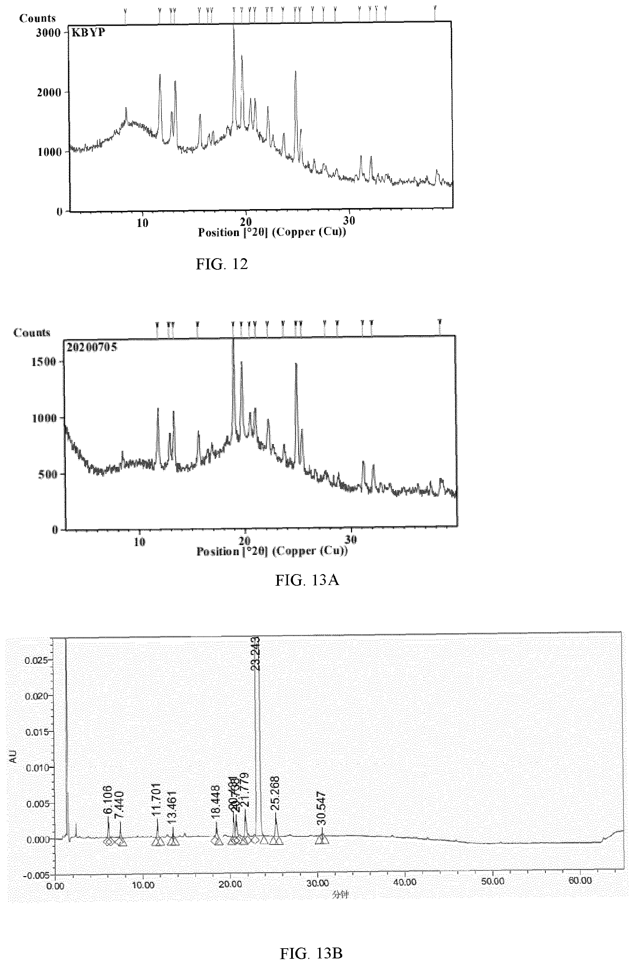

**Figure 12****:** X-ray diffraction pattern of the pharmaceutical excipients (inactive ingredients) used in the Utidelone capsules prepared in Example 5-A;
**Figure 13A****:** X-ray diffraction pattern of the contents contained in the Utidelone capsules (stored at room temperature) prepared in Example 5-A;
**Figure 13B****:** HPLC chromatogram of the contents contained in the Utidelone capsules (stored at room temperature) prepared in Example 5-A.

### Embodiments

The present application is further described below in conjunction with examples.

### Material:

Utidelone used in the formulations of the following examples was manufactured by Chengdu Huahao Zhongtian Pharmaceutical Co., Ltd., and the implementation standards of all excipients are the national approval or the 2020 edition of the Chinese Pharmacopoeia. Among them, polyoxyethylene (40) hydrogenated castor oil was made by BASF China Co., Ltd., hypromellose E50 was made by Rohm and Haas, hypromellose K100 was made by Rohm and Haas, pill cores (sucrose) were made by Shanghai Colorcon Coating, and Vacant Hypromellose Capsules were manufactured by Suzhou Capsule Co., Ltd. However, the excipients used in the oral solid formulations of the following examples are not limited by the manufacturer.

### Example 1: Utidelone oral solid formulations

| **Material** | **Amount (Formulation 1)** | **Amount (Formulation 2)** |
|---|---|---|
| Utidelone | 15g | 15g |
| Povidone K30 | 155g | 110g |
| Polyoxyethylene (40) Hydrogenated Castor Oil | 30g | 30g |
| Hypromellose E5 | 0 | 45g |
| Sugar Spheres | 100g | 100g |
| anhydrous ethanol | 2800g | 2800g |
| purified water | 1200g | 1200g |

Take the indicated amount of Utidelone, polyoxyethylene (40) hydrogenated castor oil, and povidone K30 in a beaker to be dissolved with the amount of anhydrous ethanol, and then add the amount of purified water and mix well or slowly add hypromellose E5 aqueous solution and stir for about approx. 1 hour to mix well. The hypromellose E5 aqueous solution was obtained by adding the indicated amount of hypromellose E5 to the amount of purified water while stirring to dissolve. In the multifunctional granulation and coating machine, the indicated amount of pill core was added, and coated with the above obtained solution containing drug. After coated, fully dried and discharged, and the obtained pellets were filled into capsules. Each capsule was filled with 10-30mg of Utidelone.

### Dissolution Test:

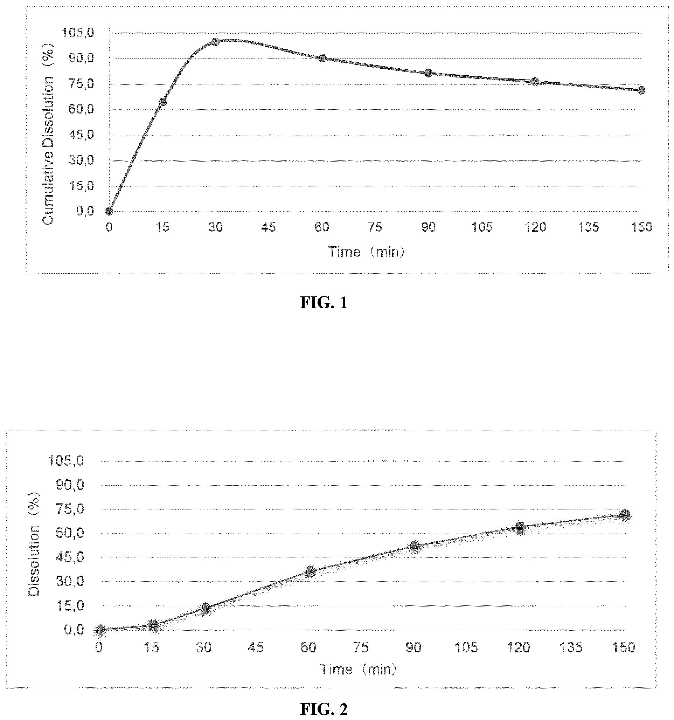

The test sample was capsules containing 20 mg of Utidelone. According to "Chinese Pharmacopoeia" 2020 Edition Appendix 0931 Dissolution and Release Determination Method 1, capsules were added to a rotating basket at a rotation speed of 100 rpm, in the dissolution medium of 900ml phosphate buffer with pH 6.8. The release was determined by high performance liquid chromatography, with a C18 chromatographic column, acetonitrile: water=(65:35) as the mobile phase, at 30 °C of column temperature, detected at a wavelength of 250 nm. The dissolution profile is shown in Figure 1.

Using the hydrophilic carrier material as a solid dispersant, after the carrier and Utidelone were prepared into a solid dispersion, although the solubility of the drug was significantly improved, the dissolved drug was prone to recrystallization in the body, thereby reducing the bioavailability of the drug. The bioavailability in the beagle of this example was 29%.

### Example 2: Utidelone oral solid formulations

| **Material** | **Amount** |
|---|---|
| Utidelone | 15g |
| Polyoxyethylene (40) Hydrogenated Castor Oil | 30g |
| Hypromellose E5 | 155g |
| Sugar Spheres | 100g |
| Ethyl cellulose | 11g |
| polyethylene glycol 400 | 15g |
| anhydrous ethanol | 2800g |
| purified water | 1200g |

Take the indicated amount of Utidelone and polyoxyethylene (40) hydrogenated castor oil in a beaker to be dissolved with the amount of anhydrous ethanol and then slowly add hypromellose E5 aqueous solution and stir for about approx. 1 hour to mix well. The hypromellose E5 aqueous solution was obtained by adding the indicated amount of hypromellose E5 to the amount of purified water while stirring till dissolved. In the multifunctional granulation and coating machine, the indicated amount of pill core was added, and coated with the above solution obtained. After coated, fully dried and discharged to render pellets containing the drug.

Take 11 g of ethyl cellulose and 1.5 g of polyethylene glycol 400 into a beaker to dissolve with 175 ml of absolute ethanol, and then add water to dilute to 250 ml to obtain a controlled release coating solution. The drug-containing pellets were coated with the controlled-release coating solution in fluidized bed, and the coating weight increased by about 4%. After coated, age at 40 °C for more than 2 hours to obtain Utidelone sustained-release pellets which were then filled in capsules.

### Dissolution Test:

The test sample was capsules containing 20 mg of Utidelone. According to "Chinese Pharmacopoeia" 2020 Edition Appendix 0931 Dissolution and Release Determination Method 1, capsules were put into a rotating basket at a rotation speed of 100 rpm, in the dissolution medium of 900ml phosphate buffer with pH 6.8. The release was determined by high performance liquid chromatography, with a C18 chromatographic column, acetonitrile: water = (65:35) as the mobile phase, at 30 °C of column temperature, detected at a wavelength of 250 nm. The dissolution profile is shown in Figure 2.

### Example 3:

Utidelone oral solid formulations

| **Material** | **Amount** |
|---|---|
| Utidelone | 15g |
| Polyoxyethylene (40) Hydrogenated Castor Oil | 30g |
| Hypromellose E50 | 80g |
| Hypromellose K100 | 25g |
| Sugar Spheres | 100g |
| anhydrous ethanol | 3000g |
| purified water | 1400g |

Take the indicated amount of Utidelone and polyoxyethylene (40) hydrogenated castor oil in a beaker to be dissolved with the amount of anhydrous ethanol and then slowly add hypromellose aqueous solution and stir for about approx. 1 hour to mix well. The hypromellose aqueous solution was obtained by adding the indicated amount of hypromellose E50 and hypromellose K100 to the amount of purified water while stirring till dissolved. In the multifunctional granulation and coating machine, the indicated amount of pill core was added, and coated with the above solution obtained. After coated, fully dried and discharged to render pellets containing the drug.

### Dissolution Test:

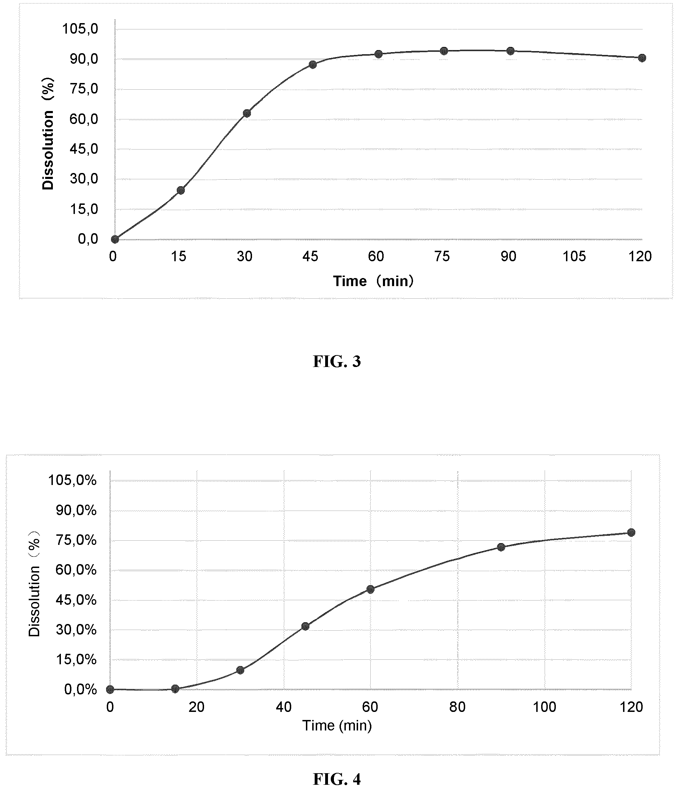

The test sample was capsules containing 20 mg of Utidelone. According to "Chinese Pharmacopoeia" 2020 Edition Appendix 0931 Dissolution and Release Determination Method 1, capsules were put into a rotating basket at a rotation speed of 100 rpm, in the dissolution medium of 900ml phosphate buffer with pH 6.8. The release was determined by high performance liquid chromatography, with a C18 chromatographic column, acetonitrile: water = (65:35) as the mobile phase, at 30 °C of column temperature, detected at a wavelength of 250 nm. The dissolution profile is shown in Figure 3.

### Example 4:

Utidelone oral solid formulations

| **Material** | **Amount** |
|---|---|
| Utidelone | 15g |
| Polyoxyethylene (40) Hydrogenated Castor Oil | 30g |
| Hypromellose E50 | 95g |
| Ethyl cellulose | 10g |
| Sugar Spheres | 100g |
| anhydrous ethanol | 2800g |
| purified water | 1200g |

Take the indicated amount of Utidelone and polyoxyethylene (40) hydrogenated castor oil and ethyl cellulose in a beaker to be dissolved with the amount of anhydrous ethanol and then slowly add hypromellose aqueous solution and stir for about approx. 1 hour to mix well. The hypromellose aqueous solution was obtained by adding the indicated amount of hypromellose E50 to the amount of purified water while stirring till dissolved. In the multifunctional granulation and coating machine, the indicated amount of pill core was added, and coated with the above solution obtained. After coated, fully dried and discharged to render pellets containing the drug, which were then filled into capsules.

### Dissolution Test:

The test sample was capsules containing 20 mg of Utidelone. According to "Chinese Pharmacopoeia" 2020 Edition Appendix 0931 Dissolution and Release Determination Method 1, capsules were put into a rotating basket at a rotation speed of 100 rpm, in the dissolution medium of 900ml phosphate buffer with pH 6.8. The release was determined by high performance liquid chromatography, with a C18 chromatographic column, acetonitrile: water = (65:35) as the mobile phase, at 30 °C of column temperature, detected at a wavelength of 250 nm. The dissolution profile is shown in Figure 4.

### Example 5-A

Utidelone oral solid formulations

| **Material** | **Amount** |
|---|---|
| Utidelone | 15g |
| Polyoxyethylene (40) Hydrogenated Castor Oil | 30g |
| Hypromellose E50 | 60g |
| Hypromellose K100 | 45g |
| Sugar Spheres | 100g |
| anhydrous ethanol * | 3000g |
| purified water * | 1400g |
| Vacant Hypromellose Capsules | as needed |

| | |
|---|---|
| *: Solvent used in the process and finally removed. | |

Take the indicated amount of Utidelone and polyoxyethylene (40) hydrogenated castor oil and ethyl cellulose in a beaker to be dissolved with the amount of anhydrous ethanol and then slowly add hypromellose aqueous solution and stir for about approx. 1 hour to mix well. The hypromellose aqueous solution was obtained by adding the indicated amount of hypromellose E50 and K100 to the amount of purified water while stirring till dissolved. In the multifunctional granulation and coating machine, the indicated amount of pill core was added, and coated with the above solution obtained. After coated, fully dried and discharged to render pellets containing the drug, which were then filled into capsules.

### Dissolution Test:

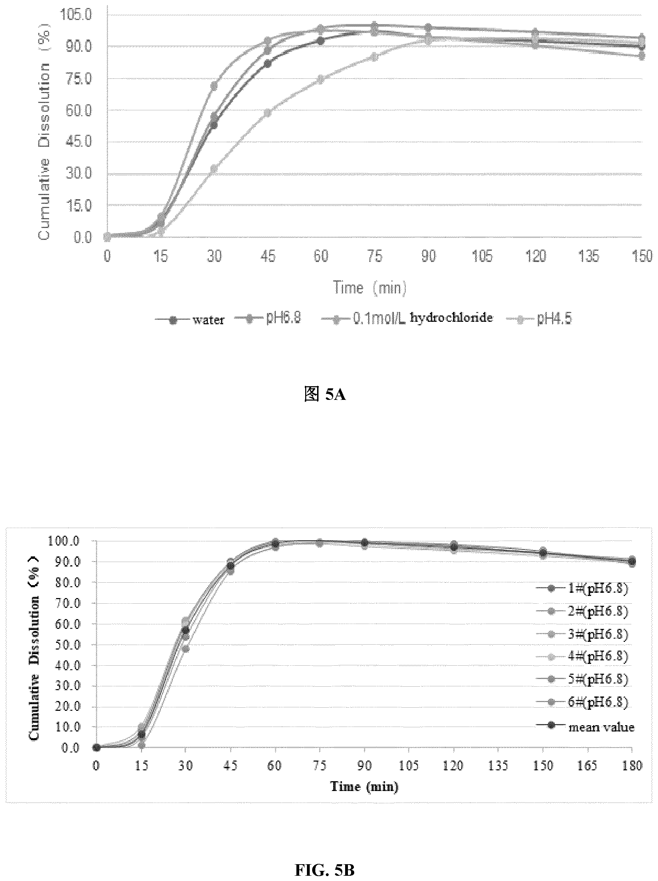

The test sample was capsules containing 20 mg of Utidelone. According to "Chinese Pharmacopoeia" 2020 Edition Appendix 0931 Dissolution and Release Determination Method 1, capsules were put into a rotating basket at a rotation speed of 100 rpm, in the dissolution medium of 900ml phosphate buffer with pH 6.8. The release was determined by high performance liquid chromatography, with a C18 chromatographic column, acetonitrile: water = (65:35) as the mobile phase, at 30 °C of column temperature, detected at a wavelength of 250 nm. The dissolution profile is shown in Figure 5A. The in vitro dissolution characteristics of the prepared samples meet the expected goals. They were dissolved about 57% at 30 minutes and about 99% at 60 minutes, and there was no burst or incomplete release occurred.

### Example 5-B

The preparation method and dissolution testing method of the micropellet capsule in this example are the same as those in Example 5-A.

| **Material** | **Amount** |
|---|---|
| Utidelone | 15g |
| Polyoxyethylene (40) Hydrogenated Castor Oil | 30g |
| Hypromellose E50 | 80g |
| Hypromellose K100 | 30g |
| Sugar Spheres | 100g |
| anhydrous ethanol * | 2500g |
| purified water * | 2000g |
| Vacant Hypromellose Capsules | as needed |

The dissolution profiles are shown in Figure 5B, showing good release uniformity for capsules prepared in multiple batches (as shown in Figure 5B).

### Example 6

Utidelone oral solid formulations

| **Material** | **Amount** |
|---|---|
| Utidelone | 15g |
| Polyoxyethylene (40) Hydrogenated Castor Oil | 30g |
| Hypromellose E50 | 60g |
| Hypromellose K100 | 45g |
| Sugar Spheres | 75g |
| Povidone K30 | 50g |
| lactose | 125g |
| talcum powder | 5g |
| anhydrous ethanol | 3000g |
| purified water | 1400g |

Take the indicated amount of Utidelone and polyoxyethylene (40) hydrogenated castor oil in a beaker to be dissolved with the amount of anhydrous ethanol and then slowly add hypromellose aqueous solution and stir for about approx. 1 hour to mix well. The hypromellose aqueous solution was obtained by adding the indicated amount of hypromellose E50 and K100 to the amount of purified water while stirring till dissolved. In the multifunctional granulation and coating machine, the indicated amount of pill core was added, and coated with the above solution obtained. After coated, fully dried and discharged to render pellets containing the drug. Mix the pellets with povidone K30, lactose and talc powder evenly, and press into tablets to obtain Utidelone tablets.

### Example 7

Utidelone oral solid formulations

| **Material** | **Amount** |
|---|---|
| Utidelone | 15g |
| Polyoxyethylene (40) Hydrogenated Castor Oil | 10g |
| Povidone K30 | 120g |
| lactose | 100g |
| Hypromellose K100M | 150g |
| talcum powder | 5g |
| anhydrous ethanol | 600g |

Take the indicated amount of Utidelone, polyoxyethylene (40) hydrogenated castor oil and povidone K30 in a beaker to be dissolved with the amount of anhydrous ethanol and mix evenly to obtain a solution containing Utidelone. The solution was spray-dried with a multifunctional granulation coating machine to obtain a solid dispersion of Utidelone. After dry granulation of the solid dispersion, lactose, hypromellose K100M and talc powder, tablet pressing was performed to obtain the Utidelone tablets.

### Example 8

Utidelone oral solid formulations

| **Material** | **Amount** |
|---|---|
| Utidelone | 15g |
| polyethylene glycol 6000 | 155g |
| Tween 80 | 30g |
| Sugar Spheres | 100g |
| anhydrous ethanol | 1500g |
| purified water | 500g |

Take the indicated amount of Utidelone, polyoxyethylene (40) hydrogenated castor oil, Tween 80, and polyethylene glycol 6000 in a beaker to dissolve with the amount of absolute ethanol, then add purified water, and mix well. In the multifunctional granulation and coating machine, the indicated amount of pill cores was added, and coated with the above solution obtained. After coated, fully dried and discharged to render pellets containing the drug, which were then filled into capsules.

### Example 9

Utidelone oral solid formulations

| **Material** | **Amount** |
|---|---|
| Utidelone | 15g |
| Poloxamer 407 | 65g |
| polyethylene glycol 8000 | 85g |
| Sugar Spheres | 100g |
| anhydrous ethanol | 1500g |
| purified water | 500g |

Take the indicated amount of Utidelone, Poloxamer 407, and polyethylene glycol 8000 in a beaker to dissolve with the amount of absolute ethanol, then add purified water, and mix well. In the multifunctional granulation and coating machine, the indicated amount of pill cores was added, and coated with the above solution obtained. After coated, fully dried and discharged to render pellets containing the drug, which were then filled into capsules.

### Example 10

Utidelone oral solid formulations

| **Material** | **Amount** |
|---|---|
| Utidelone | 15g |
| polyethylene glycol 6000 | 155g |
| Polyoxyethylene (40) Hydrogenated Castor Oil | 30g |
| Povidone K90 | 15g |
| lactose | 100g |
| silica | 155g |
| anhydrous ethanol | 1000g |

Take the indicated amount of Utidelone, polyoxyethylene (40) hydrogenated castor oil and polyethylene glycol 6000 in a beaker to be dissolved with the amount of anhydrous ethanol and mix evenly to obtain a solution containing Utidelone. The solution was spray-dried with a multifunctional granulation coating machine to obtain a solid dispersion of Utidelone. After dry granulation of the solid dispersion, lactose, silica and povidone K90, tablet pressing was performed to obtain the Utidelone tablets.

### Example 11

Utidelone oral solid formulations

| **Material** | **Amount** |
|---|---|
| Utidelone | 15g |
| polyethylene glycol 4000 | 150g |
| Poloxamer 407 | 30g |
| Glyceryl Behenate | 30g |
| Polyvinylpyrrolidone | 5g |
| sucrose | 100g |
| Micro powder silica | 100g |
| Anhydrous ethanol | 1500g |

Take the indicated amount of Utidelone, Poloxamer 407 and polyethylene glycol 4000 in a beaker to be dissolved with the amount of anhydrous ethanol and mix evenly to obtain a solution containing Utidelone. The solution was spray-dried with a multifunctional granulation coating machine to obtain a solid dispersion of Utidelone. After dry granulation of the solid dispersion, lactose, silica, glyceryl behenate, and polyvinylpyrrolidone, tablet pressing was performed to obtain Utidelone tablets.

### Example 12

Utidelone oral solid formulations

| **Material** | **Amount** |
|---|---|
| Utidelone | 15g |
| Polysorbate 80 | 30g |
| lactose | 110g |
| Cellulose acetate | 45g |
| pill core | 100g |
| anhydrous ethanol | 3000g |
| purified water | 1400g |

Take the indicated amount of Utidelone, polysorbate 80, and cellulose acetate in a beaker to dissolve with the amount of absolute ethanol, then add slowly lactose aqueous water obtained by dissolving lactose into the amount of purified water while stirring, and mix well by stirring for about 1 hour. In the multifunctional granulation and coating machine, the indicated amount of pill cores was added, and coated with the above solution obtained. After coated, fully dried and discharged to render pellets containing the drug, which were then filled into capsules.

### Example 13: Bioavailability Test

### 1. Bioavailability of Utidelone Capsules prepared in Example 1

### Experiment Method:

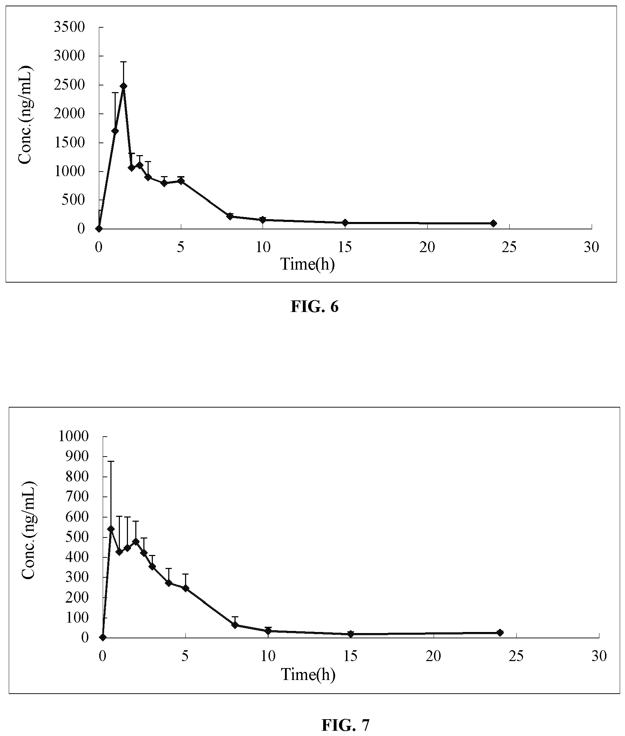

Four Beagle dogs were divided into two groups, one group was administered orally Utidelone micropellets 1mg/kg in the first test; in the other group, each dog was administered by intravenous infusion 1mg/kg of Utidelone injection in the first test. Intravenous blood was collected at 0.5h, 1h, 1.5h, 2h, 2.5h, 3h, 4h, 6h, 8h, 12h, 24h before administration and after administration. After the samples were centrifuged to separate the plasma, the concentration of Utidelone in the plasma was determined by LC-MS/MS. The test results are shown in Figure 6.

The pharmacokinetic data of Utidelone Injection and Utidelone Capsules (Example 1) are shown in the following table.

**Table 1: PK/PD data of Utidelone injection**

| **PK parameters** | **Unit** | **First Time** | | **Second Time** | | **Mean** |
|---|---|---|---|---|---|---|
| | | **101#** | **102#** | **111#** | **112#** | |
| Kₑₗ | h-1 | 0.15 | 0.06 | 0.11 | 0.14 | 0.12 |
| t_{1/2} | h | 4.48 | 11.46 | 6.21 | 5.01 | 6.79 |
| Tmax | h | 1.5 | 1.5 | 1.5 | 1.5 | 1.500 |
| Cmax | ng·mL⁻¹ | 2320 | 1590 | 2900 | 3090 | 2475 |
| AUC₀₋ₜ | ng·h·mL⁻¹ | 7964.9 | 6526.3 | 10780.5 | 10327.3 | 8899.7 |
| AUC_{0-inf} | ng·h·mL⁻¹ | 8352.6 | 7435.9 | 12195.5 | 11031.8 | 9753.9 |
| AUMC₀₋ₜ | ng·h²·mL⁻¹ | 37593.0 | 37436.3 | 68136.5 | 54171.7 | 49334.4 |
| AUMC_{0-inf} | ng·h²·mL⁻¹ | 49405.1 | 74313.1 | 114769.0 | 76174.5 | 78665.4 |
| MRT_{IV} | h | 5.91 | 9.99 | 9.41 | 6.91 | 8.06 |
| CL | mL·kg⁻¹·h⁻¹ | 119.7 | 134.5 | 82.0 | 90.6 | 106.7 |
| Vdₛₛ | mL·kg⁻¹ | 773.7 | 2224.2 | 734.3 | 655.6 | 1097.0 |

**Table 2: PK/PD data of Utidelone capsules (Example 1)**

| **PK parameters** | **Unit** | **Second Time** | | **First Time** | | **Mean** |
|---|---|---|---|---|---|---|
| | | **101#** | **102#** | **111#** | **112#** | **Mean** |
| Kₑₗ | h⁻¹ | 0.15 | 0.20 | 0.19 | 0.12 | 0.16 |
| t_{1/2} | h | 4.48 | 3.53 | 3.69 | 5.84 | 4.39 |
| Tmax | h | 1 | 0.5 | 0.5 | 2 | 1.000 |
| Cmax | ng·mL⁻¹ | 439.0 | 577.0 | 998.0 | 456.0 | 618 |
| AUC₀₋ₜ | ng·h·mL⁻¹ | 1985.3 | 2511.7 | 3463.7 | 2806.9 | 2691.9 |
| AUC_{0-inf} | ng·h·mL⁻¹ | 2134.7 | 2575.9 | 3587.6 | 3126.9 | 2856.3 |
| AUMC₀₋ₜ | ng·h²·m L⁻¹ | 9707.6 | 10242.5 | 13860.4 | 18067.8 | 12969.6 |
| AUMC_{0- inf} | ng·h²·m L⁻¹ | 14261.0 | 12111.9 | 17493.1 | 28442.4 | 18077.1 |
| MRT_{PO} | h | 4.89 | 4.08 | 4.00 | 6.44 | 4.85 |
| F | % | 21.9 | 26.4 | 36.8 | 32.1 | 29.3 |

The average relative bioavailability of the oral formulation of Example 1 can reach about 29%.

Using the same test method, the average relative bioavailability of the oral formulations prepared in Examples 2, 3, and 4 was found to be between 30-50%.

### 2. The bioavailability test of the oral formulations prepared in Examples 5 and 6

### Experimental Method:

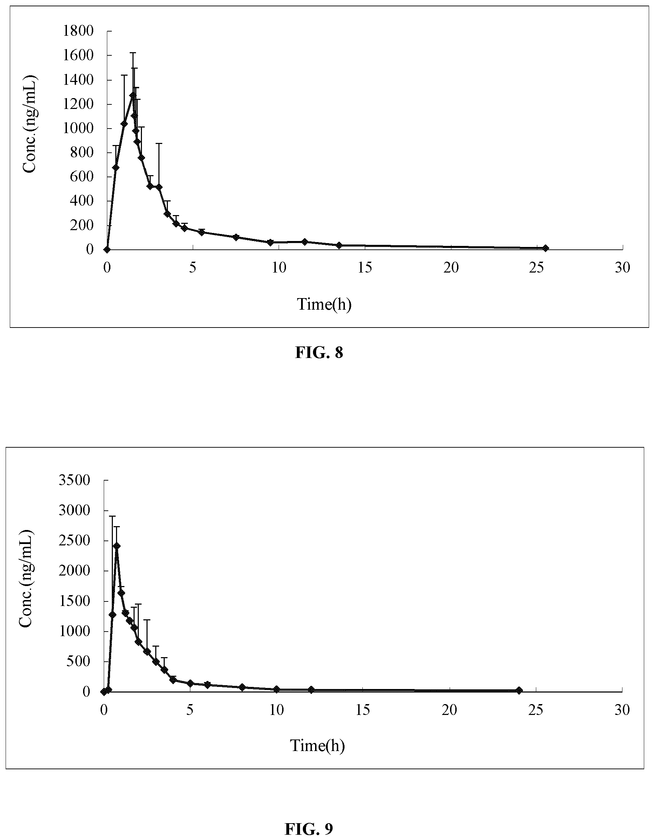

Six Beagle dogs were divided into two groups, with 3 dogs in each group. In the first group, each dog was orally administered with 1.5 mg/kg of Utidelone capsules of Example 5-A. Each dog in the other group received an intravenous infusion of 1 mg/kg of Utidelone as reference. Intravenous blood was collected at 0.5h, 1h, 1.5h, 2h, 2.5h, 3h, 4h, 6h, 8h, 12h, 24h before administration and after administration. After the samples were centrifuged to separate the plasma, the concentration of Utidelone in the plasma was determined by LC-MS/MS. The results of the injection test are shown in Figure 8, and the experimental results of the capsules are shown in FIG. 9.

The pharmacokinetic data of Utidelone injection and Utidelone capsules (Example 5-A) are shown in Table 3 below.

**Table 3: Mean values of pharmacokinetic parameters after a single intravenous administration of Utidelone injection and oral administration of Utidelone capsules in beagle dogs.**

| **Parameters** | **Mean values** | |
|---|---|---|
| | **intravenous administration** | **oral administration SD** |
| Kel (h⁻¹) | 0.12 ± 0.04 | 0.16 ± 0.13 |
| T_{1/2} (h) | 6.18 ± 2.57 | 8.70 ± 6.70 |
| Tₘₐₓ (h) | 1.5 ± 0.00 | 0.75 ± 0.43 |
| Cₘₐₓ (ng/mL) | 1273 ± 351 | 2410 ± 1331 |
| AUC₀₋ₜ | 3686 ± 733 | 4166 ± 2164 |
| AUC_{0-∞} | 3789 ± 653 | 4459 ± 2298 |
| AUMC₀₋ₜ | 15252 ± 373 | 12665 ± 5968 |
| AUMC_{0-inf} | 19017 ± 3458 | 24715 ± 16308 |
| MRT (h) | 4.47 ± 1.87 | 4.89 ± 5.88 |
| CL (mL·h) | 2694 ± 474 | - |
| Vdₛₛ (mL) | 25031 ± 14804 | - |

The relative bioavailability of Utidelone capsules after a single oral administration ranged from 53.1% to 103.8%, with an average relative bioavailability of 78.5%.

With the test method of Example 5-A, it was measured that the average relative bioavailability of the Utidelone tablets prepared in Example 6 reached more than 55%.

### Example 14: Stability

Utidelone capsules (Examples 1 and 5) were stored under the accelerated test conditions of 40°C and 75% RH, and the related substance detection results after 1 month of storage showed that the degradation impurities in the product were within the range specified by ICH Q3. It shows that the oral formulation of Utidelone of the present invention has good stability.

Utidelone capsules (Examples 1 and 5) were stored under the accelerated test conditions of 40°C and 75% RH. The X-ray diffraction analysis of the preparations after 1 month of storage showed that the Utidelone in the capsules exists in an amorphous or molecular form.

### Example 15: Preparation of Amorphous Utidelone

### Method 1:

Dissolve 1 g of Utidelone in 5 ml of dichloromethane or chloroform or any two or three mixed solvents of dichloromethane, chloroform and ethyl acetate, and dried under reduced pressure of -0.05Mpa at 30°C~80°Cto obtain the target product.

### Method 2:

Dissolve 1g of Utidelone in 5ml of dichloromethane or chloroform or any two or three mixed solvents of dichloromethane, chloroform and ethyl acetate, and spray-dry it with a fluidized bed with feed and inlet air temperature of ≥30°C to obtain the target product.

### Method 3:

Dissolve 1 g of Utidelone in 10 ml of methanol or ethanol, and evaporate to dryness under reduced pressure with a rotary evaporator to obtain the target product.

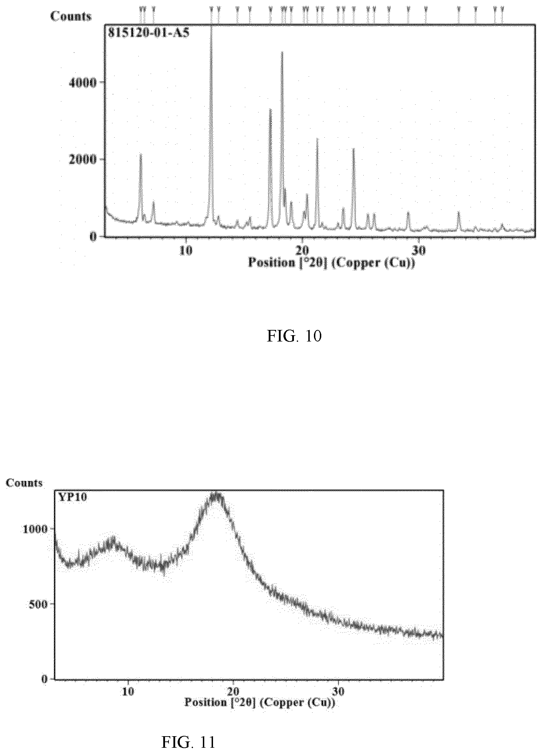

The X-ray diffraction pattern of amorphous Utidelone is shown in FIG. 11 .

Utidelone in the capsules obtained in the examples of the present application exists in an amorphous or molecular form.

## Claims

1. A solid oral formulation comprising Utidelone, or a pharmaceutically acceptable salt, solvate or ester thereof as an active ingredient and a pharmaceutically acceptable excipient, wherein the formulation is a micropellet capsule encapsulating micropellets, or a tablet prepared by micropellet compression, wherein the micropellets are pellet cores coated with a coating layer comprising the active ingredient and said pharmaceutically acceptable excipient,
wherein the pharmaceutically acceptable excipient comprises a hydrophilic pharmaceutical excipient, a sustained-release pharmaceutical excipient, and optionally a surfactant, and
wherein the ratio of the active ingredient to the pharmaceutically acceptable excipient is in the range of 1:1 to 1:30.

2. The solid oral formulation according to claim 1, wherein the ratio of the active ingredient to the pharmaceutically acceptable excipient is in the range of 1:5 to 1:20.

3. The solid oral formulation according to any of claims 1 to 2, wherein the hydrophilic pharmaceutical excipient is selected from at least one of povidone, hypromellose, mannitol, lactose, sucrose, poloxamer and polyvinyl alcohol; the sustained-release pharmaceutical excipient is selected from at least one of povidone, hypromellose, polyethylene glycol, ethyl cellulose, polyvinyl acetal diethylamine acetate, hypromellose acetate succinate, acetate methacrylate copolymer, cellulose acetate, methyl cellulose, polyacrylic resin, polyvinyl phthalate, cellulose phthalate, and hypromellose phthalate; the surfactant is selected from at least one of polysorbate, polyoxyl castor oil, sodium lauryl sulfate, cholate, fatty acid glyceride, sorbitan, polyoxyethylene fatty acid ester, polyoxyethylene fatty alcohol ether, and poloxamer.

4. The solid oral formulation according to claim 3, wherein the hydrophilic pharmaceutical excipient is selected from at least one of low viscosity hypromellose, povidone and poloxamer; the sustained-release pharmaceutical excipient is selected from at least one of high viscosity hypromellose, high viscosity polyethylene glycol, ethyl cellulose and cellulose acetate; the surfactant is selected from at least one of polyoxyl castor oil, polysorbate and poloxamer.

5. The solid oral formulation according to any of claims 1-4, wherein the micropellet capsule or tablet comprises 2%-10% (w/w) of the active ingredient based on the weight of the micropellet or the weight of the tablet.

6. The solid oral formulation according to claim 5, wherein the micropellet capsule or tablet further comprises 30%-70% (w/w) of pharmaceutically acceptable excipients and 20%~60% (w/w) pellet core based on the weight of the micropellet or tablet.

7. The solid oral formulation according to any of claims 1-6, which is a micropellet capsule comprising Utidelone as an active ingredient, and polyoxyethylene (40) hydrogenated castor oil, low-viscosity hypromellose and high viscosity hypromellose as pharmaceutically acceptable excipients.

8. The solid oral formulation according to any of claims 1-7, wherein the active ingredient Utidelone is present in an amorphous or molecular form.

9. The solid oral formulation according to any of claims 1-8 for use in a method of treating a cancer selected from breast cancer, lung cancer, digestive tract tumors, lymphoid tumors, prostate cancer, brain cancer, gynecological tumors, liver cancer, head and neck tumors, ovarian cancer, colon cancer and stomach cancer.

## Patentansprüche

1. Feste orale Formulierung, umfassend Utidelon oder ein pharmazeutisch verträgliches Salz, Solvat oder Ester davon als Wirkstoff und einen pharmazeutisch verträglichen Hilfsstoff, wobei die Formulierung eine Mikropelletkapsel, die Mikropellets einkapselt, oder eine Tablette ist, die durch Mikropelletkompression hergestellt wird, wobei die Mikropellets Pelletkerne sind, die mit einer Überzugsschicht beschichtet sind, die den Wirkstoff und den pharmazeutisch verträglichen Hilfsstoff umfasst,
wobei der pharmazeutisch verträgliche Hilfsstoff einen hydrophilen pharmazeutischen Hilfsstoff, einen pharmazeutischen Hilfsstoff mit verzögerter Freisetzung und gegebenenfalls ein Tensid umfasst, und
wobei das Verhältnis des Wirkstoffs zu dem pharmazeutisch verträglichen Hilfsstoff im Bereich von 1:1 bis 1:30 liegt.

2. Feste orale Formulierung nach Anspruch 1, wobei das Verhältnis des Wirkstoffs zum pharmazeutisch verträglichen Hilfsstoff im Bereich von 1:5 bis 1:20 liegt.

3. Feste orale Formulierung nach einem der Ansprüche 1 bis 2, wobei der hydrophile pharmazeutische Hilfsstoff aus mindestens einem von Povidon, Hypromellose, Mannitol, Lactose, Saccharose, Poloxamer und Polyvinylalkohol ausgewählt ist; der pharmazeutische Hilfsstoff mit verzögerter Freisetzung aus mindestens einem von Povidon, Hypromellose, Polyethylenglykol, Ethylcellulose, Polyvinylacetaldiethylaminacetat, Hypromelloseacetatsuccinat, Acetatmethacrylat-Copolymer, Celluloseacetat, Methylcellulose, Polyacrylharz, Polyvinylphthalat, Cellulosephthalat und Hypromellosephthalat ausgewählt ist; das Tensid aus mindestens einem von Polysorbat, Polyoxyl-Rizinusöl, Natriumlaurylsulfat, Cholat, Fettsäureglycerid, Sorbitan, Polyoxyethylen-Fettsäureester, Polyoxyethylen-Fettalkoholether und Poloxamer ausgewählt ist.

4. Feste orale Formulierung nach Anspruch 3, wobei der hydrophile pharmazeutische Hilfsstoff aus mindestens einem von Hypromellose mit niedriger Viskosität, Povidon und Poloxamer ausgewählt ist; der pharmazeutische Hilfsstoff mit verzögerter Freisetzung aus mindestens einem von Hypromellose mit hoher Viskosität, Polyethylenglykol mit hoher Viskosität, Ethylcellulose und Celluloseacetat ausgewählt ist; das Tensid aus mindestens einem von Polyoxyl-Rizinusöl, Polysorbat und Poloxamer ausgewählt ist.

5. Feste orale Formulierung nach einem der Ansprüche 1-4, wobei die Mikropelletkapsel oder -tablette 2 % -10 % (Gw/Gw) des Wirkstoffs umfasst, basierend auf dem Gewicht des Mikropellets oder dem Gewicht der Tablette.

6. Feste orale Formulierung nach Anspruch 5, wobei die Mikropelletkapsel oder -tablette ferner 30%-70% (Gw/Gw) pharmazeutisch verträgliche Hilfsstoffe und 20%~60% (Gw/Gw) Pelletkern basierend auf dem Gewicht des Mikropellets oder der Tablette umfasst.

7. Feste orale Formulierung nach einem der Ansprüche 1-6, bei der es sich um eine Mikropelletkapsel handelt, die Utidelon als Wirkstoff und Polyoxyethylen (40)-hydriertes Rizinusöl, niedrigviskose Hypromellose und hochviskose Hypromellose als pharmazeutisch verträgliche Hilfsstoffe umfasst.

8. Feste orale Formulierung nach einem der Ansprüche 1-7, wobei der Wirkstoff Utidelon in einer amorphen oder molekularen Form vorliegt.

9. Feste orale Formulierung nach einem der Ansprüche 1-8 zur Verwendung in einem Verfahren zum Behandeln einer Krebserkrankung, ausgewählt aus Brustkrebs, Lungenkrebs, Tumoren des Verdauungstrakts, lymphatischen Tumoren, Prostatakrebs, Hirnkrebs, gynäkologischen Tumoren, Leberkrebs, Kopf- und Halstumoren, Eierstockkrebs, Dickdarmkrebs und Magenkrebs.

## Revendications

1. Formulation orale solide comprenant de l'utidélone, ou un sel, un solvate ou un ester pharmaceutiquement acceptable de celle-ci en tant que principe actif et un excipient pharmaceutiquement acceptable, dans laquelle la formulation est une capsule de microgranules encapsulant des microgranules, ou un comprimé préparé par compression de microgranules, dans laquelle les microgranules sont des noyaux de granules enrobés d'une couche d'enrobage comprenant le principe actif et ledit excipient pharmaceutiquement acceptable,
dans laquelle l'excipient pharmaceutiquement acceptable comprend un excipient pharmaceutique hydrophile, un excipient pharmaceutique à libération prolongée, et éventuellement un tensioactif, et
dans laquelle le rapport entre le principe actif et l'excipient pharmaceutiquement acceptable est dans la plage de 1:1 à 1:30.

2. Formulation orale solide selon la revendication 1, dans laquelle le rapport entre le principe actif et l'excipient pharmaceutiquement acceptable est dans la plage de 1:5 à 1:20.

3. Formulation orale solide selon l'une quelconque des revendications 1 et 2, dans laquelle l'excipient pharmaceutique hydrophile est choisi parmi l'au moins un de la povidone, de l'hypromellose, du mannitol, du lactose, du saccharose, du poloxamère et de l'alcool polyvinylique; l'excipient pharmaceutique à libération prolongée est choisi parmi l'au moins un de la povidone, de l'hypromellose, du polyéthylène glycol, de l'éthylcellulose, de l'acétate de polyvinyl-acétal-diéthylamine, du succinate d'acétate d'hypromellose, du copolymère acétate-méthacrylate, de l'acétate de cellulose, de la méthylcellulose, de la résine polyacrylique, du phtalate de polyvinyle, du phtalate de cellulose et du phtalate d'hypromellose ; le tensioactif est choisi parmi l'au moins un du polysorbate, de l'huile de ricin polyoxylée, du laurylsulfate de sodium, du cholate, du glycéride d'acide gras, du sorbitan, de l'ester d'acide gras de polyoxyéthylène, de l'éther d'alcool gras de polyoxyéthylène et du poloxamère.

4. Formulation orale solide selon la revendication 3, dans laquelle l'excipient pharmaceutique hydrophile est choisi parmi l'au moins un de l'hypromellose à faible viscosité, de la povidone et du poloxamère ; l'excipient pharmaceutique à libération prolongée est choisi parmi l'au moins un de l'hypromellose à haute viscosité, du polyéthylène glycol à haute viscosité, de l'éthylcellulose et de l'acétate de cellulose ; le tensioactif est choisi parmi l'au moins un de l'huile de ricin polyoxylée, du polysorbate et du poloxamère.

5. Formulation orale solide selon l'une quelconque des revendications 1 à 4, dans laquelle la capsule ou le comprimé de microgranules comprend 2 % à 10 % (p/p) du principe actif sur la base du poids de la microgranule ou du poids du comprimé.

6. Formulation orale solide selon la revendication 5, dans laquelle la capsule ou le comprimé de microgranules comprend en outre 30 % à 70 % (p/p) d'excipients pharmaceutiquement acceptables et environ 20 % à 60 % (p/p) de noyau de microgranules par rapport au poids de la microgranule et du comprimé.

7. Formulation orale solide selon l'une quelconque des revendications 1 à 6, qui est une capsule de microgranules comprenant de l'utidélone comme principe actif, et de l'huile de ricin hydrogénée de polyoxyéthylène (40), de l'hypromellose de faible viscosité et de l'hypromellose de forte viscosité comme excipients pharmaceutiquement acceptables.

8. Formulation orale solide selon l'une quelconque des revendications 1 à 7, dans laquelle le principe actif utidélone est présent sous forme amorphe ou moléculaire.

9. Formulation orale solide selon l'une quelconque des revendications 1 à 8, destinée à être utilisée dans un procédé de traitement d'un cancer choisi parmi le cancer du sein, le cancer du poumon, les tumeurs du tube digestif, les tumeurs lymphoïdes, le cancer de la prostate, le cancer du cerveau, les tumeurs gynécologiques, le cancer du foie, les tumeurs de la tête et du cou, le cancer des ovaires, le cancer du côlon et le cancer de l'estomac.
